# FASCICULE DE BREVET EUROPEEN

(11) **EP 3 685 398 B1**
(45) Date de publication et mention de la délivrance du brevet: **27.03.2024**
(21) Numéro de dépôt: 18786838.5
(22) Date de dépôt: 21.09.2018
(51) Int. Cl.: G16H 40/63, G16H 40/67, A61N 1/36, A61N 1/372

(54) **SYSTEME DE COMMUNICATION TELEMETRIQUE POUR UN DISPOSITIF MEDICAL IMPLANTABLE**
TELEMETRISCHES KOMMUNIKATIONSSYSTEM FÜR EINE IMPLANTIERBARE MEDIZINISCHE VORRICHTUNG
TELEMETRIC COMMUNICATION SYSTEM FOR AN IMPLANTABLE MEDICAL DEVICE

(30) Priorité: 22.09.2017 FR 1758771
(43) Date de publication de la demande: 29.07.2020
(73) Titulaire: Uromems, 38320 Eybens (FR)
(72) Inventeur: LAMRAOUI, Hamid, 38000 Grenoble (FR); MARIEN, Marc, 38700 La Tronche (FR); LAVALLEE, Stéphane, 38410 St Martin D'uriage (FR)
(74) Mandataire: Regimbeau
(86) Numéro de dépôt international: PCT/FR2018/052304
(87) Numéro de publication internationale: WO 2019/058064

(56) Documents cités:
- US-A1- 2002 123 673
- US-A1- 2012 035 686
- US-A1- 2016 303 313

## Description

### DOMAINE DE L'INVENTION

La présente invention concerne un système de communication télémétrique pour un dispositif médical implantable.

### ARRIERE PLAN DE L'INVENTION

Il existe de nombreux dispositifs médicaux implantables dans un patient pour remplir une fonction dans le corps du patient, par exemple pour pallier un dysfonctionnement d'un organe naturel.

Dans ce cadre, il est généralement nécessaire de pouvoir communiquer avec le dispositif implanté (également appelé implant dans la suite du texte). Cette communication peut avoir pour but de piloter le fonctionnement du dispositif implanté (par exemple pour l'activer ou le désactiver, ou pour mettre en oeuvre un mode de fonctionnement particulier), de configurer ses paramètres internes ou d'obtenir des informations de la part du dispositif (par exemple pour connaître l'état du dispositif ou pour obtenir des paramètres du patient mesurés par des capteurs implantés avec le dispositif).

A cet effet, il est connu d'équiper le patient d'une télécommande.

Par exemple, dans le cas de la neurostimulation, l'implant est constitué d'un stimulateur et d'une électrode implantés sous la peau du patient, de sorte à permettre d'appliquer des impulsions électriques à des zones déterminées de la moelle épinière. La génération des impulsions est commandée par une télécommande détenue par le patient, qui doit placer la télécommande sur la peau au voisinage du stimulateur afin d'actionner le stimulateur par induction.

Pour faciliter l'utilisation, les nouvelles générations d'implants sont équipées de puces radiofréquence (RF).

Pour permettre une communication bidirectionnelle entre ladite télécommande et le dispositif implanté, chacun d'eux comprend une puce radiofréquence (RF) comprenant à la fois un émetteur et un récepteur d'ondes RF.

Une telle télécommande permet ainsi au patient de commander certaines fonctions du dispositif implanté au quotidien, par exemple pour l'activer ou le désactiver si nécessaire. La télécommande permet également au patient d'obtenir des informations sur l'état du dispositif, par exemple l'état de charge d'une source d'énergie implantée avec le dispositif.

Cette télécommande présente toutefois des fonctionnalités limitées et ne permet pas, par exemple, de programmer le fonctionnement du dispositif implanté.

Une telle intervention sur le fonctionnement du dispositif de l'implant est quant à elle réservée au praticien.

Cette intervention peut avoir lieu par exemple au bloc opératoire, lorsque le dispositif est implanté chez le patient, pour programmer le dispositif en fonction des caractéristiques du patient.

Cette intervention peut avoir lieu par la suite lors de consultations du patient chez le praticien, pendant lesquelles le praticien récupère des données de l'implant pour les analyser et éventuellement reprogramme l'implant.

A cet effet, le praticien dispose d'un dispositif de programmation (également appelé programmateur dans la suite du texte) qui présente davantage de fonctionnalités que la télécommande du patient. Ce programmateur comprend ainsi généralement une interface permettant à un utilisateur d'entrer de nouveaux paramètres de fonctionnement du patient et de visualiser les données de l'implant.

Il est donc nécessaire d'établir une communication entre le programmateur et l'implant, ce qui implique les considérations suivantes.

En premier lieu, il faut s'assurer qu'aucun tiers non autorisé ne puisse communiquer avec l'implant, pour des raisons de sécurité du patient (prise de contrôle de l'implant par le tiers) et de respect de ses données médicales (récupérées de l'implant).

D'autre part, il faut s'assurer que le praticien communique avec le bon implant. Compte tenu de la distance de portée des émetteurs-récepteurs RF qui est typiquement de quelques mètres, si plusieurs implants se trouvent à proximité du programmateur, il existe un risque de confusion entraînant la communication avec un implant différent de celui avec lequel on souhaite communiquer.

Il existe également des contraintes en termes d'utilisation du programmateur. Le praticien doit en effet pouvoir utiliser le programmateur d'une manière simple et en un minimum d'actions.

Par ailleurs, le programmateur doit pouvoir être utilisé au bloc opératoire, pour programmer l'implant avant ou pendant l'implantation. Comme il est nécessaire de maintenir le programmateur à distance du champ stérile, une communication RF peut être dégradée compte tenu de la réflexion des ondes dans le bloc opératoire. Approcher le programmateur de l'implant nécessiterait de placer le programmateur dans une housse stérile, ce qui pose des problèmes pratiques.

US 2016/303313 A1 concerne la programmation et le contrôle du fonctionnement des dispositifs implantables à l'aide d'un dispositif informatique mobile couramment utilisé. L'objet de la revendication 1 diffère du procédé connu dans US 2016/303313 A1 en l'appairage des deux dispositifs et en l'utilisation de deux bandes de fréquence différentes (l'une dédiée à des dispositifs médicaux et l'autre à des applications de grand public). Ce document ne décrit aucun code d'appairage, a fortiori ne décrit aucune saisie manuelle par le praticien d'un code d'appairage associé au dispositif implantable et/ou d'un code d'appairage associé à la télécommande, dans l'interface utilisateur du dispositif de programmation. Ce document représente l'art antérieur le plus proche.

### BREVE DESCRIPTION DE L'INVENTION

Un but de l'invention est de remédier aux problèmes précités et de concevoir un système de communication bidirectionnelle d'un implant avec la télécommande du patient et le programmateur du praticien qui soit sécurisé et de bonne qualité.

Conformément à l'invention, il est proposé un système de communication télémétrique pour un dispositif médical implantable comprenant un émetteur-récepteur radiofréquence, comprenant :
- une télécommande adaptée pour être utilisée par un patient dans lequel ledit dispositif médical est implanté, ladite télécommande comprenant un émetteur-récepteur radiofréquence configuré pour communiquer avec ledit dispositif médical implantable dans une première bande de fréquence, l'émetteur-récepteur de la télécommande étant appairé avec l'émetteur-récepteur du dispositif médical implantable,
- un dispositif de programmation dudit dispositif médical implantable adapté pour être utilisé par un praticien, comprenant une interface utilisateur, et configuré pour communiquer avec la télécommande par une liaison filaire ou par une liaison sans fil dans une seconde bande de fréquence différente de la première bande de fréquence,
le dispositif de programmation étant configuré pour, lorsque ladite liaison est établie entre la télécommande et le dispositif de programmation, établir une communication entre le dispositif médical implantable et le dispositif de programmation par l'intermédiaire de la télécommande.

La première bande de fréquence est une bande de fréquence dédiée aux dispositifs médicaux, distincte de la bande de fréquence destinée aux applications grand public, ce qui permet d'assurer la sécurité des communications entre le dispositif médical implantable et la télécommande.

En revanche, la seconde bande de fréquence est une bande de fréquence dédiée aux applications grand public, ce qui offre une grande souplesse quant à l'établissement d'une communication entre le dispositif de programmation et la télécommande. De manière alternative, une telle souplesse peut être procurée par la mise en oeuvre d'une liaison filaire entre le dispositif de programmation et la télécommande. Dans tous les cas, la sécurité des communications entre le dispositif de programmation et le dispositif médical implantable est assurée par la télécommande qui est indispensable à l'établissement de ces communications.

Par « praticien » on entend toute personne du corps médical habilitée à programmer l'implant et/ou à consulter des données enregistrées dans l'implant, comme par exemple un chirurgien, un médecin, ou un infirmier.

Selon un mode de réalisation, le dispositif de programmation comprend un logement pour l'enfichage de la télécommande.

De manière avantageuse, le dispositif de programmation comprend un lecteur adapté pour lire un code d'appairage associé au dispositif implantable et/ou un code d'appairage associé à la télécommande.

Selon un mode de réalisation, le code d'appairage comprend un code à barres.

Le lecteur peut être de type optique et/ou communication en champ proche, par exemple de type RFID ou NFC.

Selon une forme d'exécution, la liaison entre la télécommande et le dispositif de programmation est une liaison sans fil, par exemple de type Bluetooth, Wifi ou infrarouge.

De manière alternative, la liaison entre le dispositif de programmation et la télécommande est une liaison filaire, le dispositif de programmation et la télécommande comprenant des connecteurs respectifs adaptés pour coopérer l'un avec l'autre.

Selon un mode de réalisation, chacun de la télécommande et du dispositif de programmation comprend un microcontrôleur et la liaison entre la télécommande et le dispositif de programmation comprend une transmission de données entre lesdits microcontrôleurs.

Selon un mode de réalisation, le dispositif de programmation comprend un microcontrôleur et en la liaison entre la télécommande et le dispositif de programmation comprend une transmission de données entre ledit microcontrôleur et l'émetteur-récepteur de la télécommande.

Selon un mode de réalisation, la télécommande comprend un microcontrôleur et la liaison entre la télécommande et le dispositif de programmation comprend une transmission de données entre ledit microcontrôleur et l'interface utilisateur du dispositif de programmation.

Le dispositif de programmation est avantageusement configuré pour afficher sur l'interface utilisateur des informations relatives à l'état de la télécommande lorsque la télécommande est en liaison avec ledit dispositif de programmation.

Selon une forme d'exécution, la liaison entre la télécommande et le dispositif de programmation comprend la fourniture d'énergie à la télécommande par le dispositif de programmation.

Un autre objet de l'invention concerne un procédé de communication entre un dispositif médical implantable comprenant un émetteur-récepteur radiofréquence et un dispositif de programmation d'un système tel que décrit précédemment, caractérisé en ce qu'il comprend l'établissement d'une liaison entre la télécommande dudit système et le dispositif de programmation et l'établissement d'une communication entre le dispositif de programmation et le dispositif médical implantable par l'intermédiaire de la télécommande.

Selon un mode de réalisation, ledit procédé comprend, préalablement à l'établissement de la communication entre le dispositif de programmation et le dispositif médical implantable, une étape d'appairage de la télécommande avec le dispositif médical implantable comprenant :
- la lecture d'un code d'appairage de la télécommande par le dispositif de programmation, ou la saisie dudit code sur le dispositif de programmation,
- l'établissement d'une liaison entre la télécommande et le dispositif de programmation,
- la lecture d'un code d'appairage du dispositif médical implantable par le dispositif de programmation, ou la saisie dudit code sur le dispositif de programmation, et l'envoi dudit code à la télécommande,
- la validation de l'appairage avec lesdits codes.

Selon un mode de réalisation, en l'absence de la télécommande appairée avec le dispositif médical implantable, on utilise une autre télécommande non appairée audit dispositif médical implantable et l'on met en oeuvre les étapes suivantes :
- lecture d'un code d'appairage de ladite autre télécommande par le dispositif de programmation, ou saisie dudit code sur le dispositif de programmation,
- établissement d'une liaison entre ladite autre télécommande et le dispositif de programmation,
- lecture du code d'appairage du dispositif médical implantable, ou la saisie dudit code sur le dispositif de programmation, et envoi dudit code à ladite autre télécommande,
- confirmation, par un utilisateur, d'une commande d'appairage.

Selon un mode de réalisation, en l'absence de la télécommande appairée avec le dispositif médical implantable, on utilise une autre télécommande non appairée audit dispositif médical implantable et l'on met en oeuvre les étapes suivantes :
- lecture d'un code d'appairage de ladite autre télécommande par le dispositif de programmation, ou saisie dudit code sur le dispositif de programmation,
- établissement d'une liaison entre ladite autre télécommande et le dispositif de programmation,
- détection d'au moins un dispositif médical à portée de ladite autre télécommande,
- sélection, par un utilisateur, du dispositif médical implantable avec lequel ladite autre télécommande doit être appairée,
- confirmation, par l'utilisateur, d'une commande d'appairage.

Selon une forme d'exécution particulière, la confirmation de la commande d'appairage avec le dispositif médical implantable sélectionné comprend l'exécution d'une série de tapotements selon un code prédéterminé sur la peau du patient en regard du dispositif médical implantable, ledit code étant détecté par un capteur dudit dispositif.

Selon un mode de réalisation, en l'absence de la télécommande appairée avec le dispositif médical implantable, on utilise une télécommande dite universelle, configurée pour communiquer avec n'importe quel dispositif médical implantable du même type et l'on met en oeuvre les étapes suivantes :
- lecture d'un code d'appairage de ladite télécommande universelle par le dispositif de programmation,
- établissement d'une liaison entre ladite télécommande universelle et le dispositif de programmation,
- détection d'au moins un dispositif médical à portée de ladite télécommande universelle,
- sélection, par un utilisateur, du dispositif médical implantable avec lequel la liaison doit être effectuée,
- confirmation, par l'utilisateur, de la liaison entre ledit dispositif médical implantable et le dispositif de programmation.

Selon une forme d'exécution particulière, la confirmation de la liaison entre le dispositif médical implantable sélectionné et le dispositif de programmation comprend l'exécution d'une série de tapotements selon un code prédéterminé sur la peau du patient en regard du dispositif médical implantable, ledit code étant détecté par un capteur dudit dispositif.

### BREVE DESCRIPTION DES DESSINS

D'autres caractéristiques et avantages de l'invention ressortiront de la description détaillée qui va suivre, en référence aux dessins annexés sur lesquels :
- les figures 1A à 1C sont des schémas de principe de la communication de l'implant avec le dispositif de programmation et la télécommande, selon différents modes de réalisation de l'invention,
- la figure 2 est un schéma de principe de l'architecture du système de communication selon l'invention,
- les figures 3A-3B, 4A-4B et 5A-5B illustrent trois modes de réalisation d'une liaison filaire entre la télécommande et le dispositif de programmation,
- la figure 6 illustre de manière schématique une liaison sans fil entre la télécommande et le dispositif de programmation,
- les figures 7 à 9 illustrent différents états du système de la figure 2 selon l'appairage entre l'implant et la télécommande.

Les signes de référence identiques d'une figure à l'autre désignent des composants identiques ou remplissant la même fonction.

### DESCRIPTION DETAILLEE DE MODES DE REALISATION DE L'INVENTION

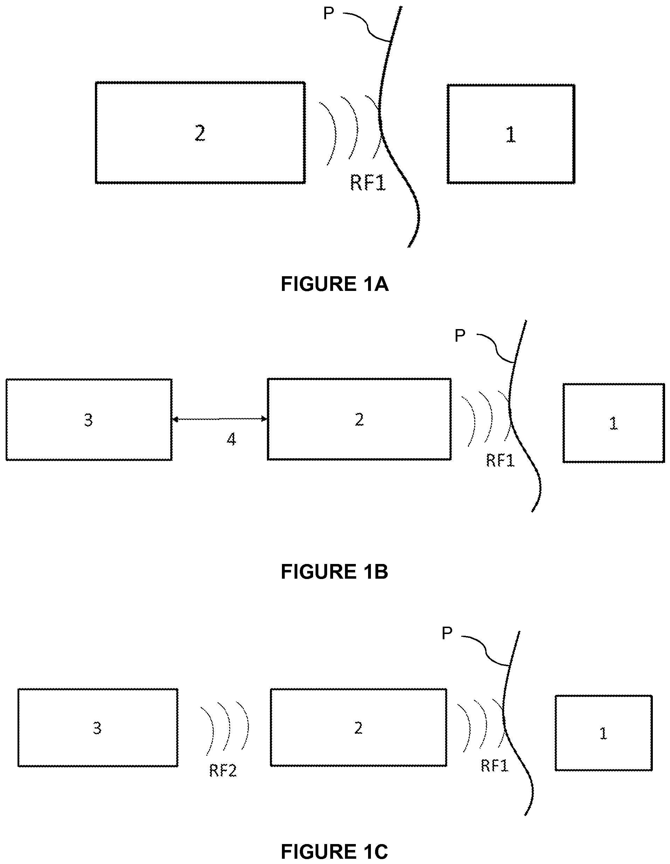

Les figures 1A à 1C sont des schémas de principe du système de communication permettant la communication de l'implant avec le dispositif de programmation et la télécommande. Sur ces figures, le patient (dont seule une partie de la peau est représentée) est désigné par le repère P.

Par « communication » on entend la transmission de données entre deux dispositifs.

Ladite communication est bidirectionnelle, c'est-à-dire qu'elle comprend la transmission de signaux de et vers l'implant, par exemple pour programmer l'implant ou récupérer des données de celui-ci.

Comme expliqué en détail plus bas, dans ce système, seule la télécommande est apte à communiquer avec l'implant par télémétrie. A cet effet, la télécommande est appairée avec l'implant avec un code spécifique, et la communication se fait dans une bande de fréquence déterminée, notée RF1 dans la suite de la description. Ladite bande de fréquence est avantageusement spécifique aux dispositifs médicaux implantables, et permet de limiter les interférences avec d'autres types de dispositifs radiofréquence (autres que des implants) lors d'échange de données entre l'implant et la télécommande. Ainsi, la bande de fréquence est avantageusement la bande MICS (acronyme du terme anglo-saxon « Medical Device Radiocommunications Service »).

Quant au dispositif de programmation, il ne peut pas communiquer directement avec l'implant mais avec la télécommande, qui relaie la communication.

La communication entre le dispositif de programmation et la télécommande peut être établie soit de manière filaire, soit par liaison sans fil. Dans le cas d'une liaison sans fil, la communication entre le dispositif de programmation et la télécommande se fait dans une bande de fréquence notée RF2 dans la suite de la description, différente de la bande de fréquence RF1. De manière avantageuse, la bande de fréquence RF2 est une bande de fréquence standard (c'est-à-dire destinée au grand public), non spécifique à l'implant.

Dans le cas où la télécommande n'est pas disponible lors d'une consultation du patient chez le praticien, la communication entre le dispositif de programmation et l'implant peut être établie comme suit.

Le praticien peut disposer d'une télécommande dite « universelle », c'est-à-dire similaire à la télécommande du patient mais non appairée avec celui-ci, et apte à communiquer avec le dispositif de programmation et avec n'importe quel implant du même type que celui implanté dans le patient.

Afin d'établir une communication avec l'implant et de s'assurer que le praticien communique avec le bon implant, une procédure de vérification doit être mise en oeuvre. Par exemple, avant une session de communication, le praticien doit vérifier le numéro de série de l'implant et/ou entrer un mot de passe. Eventuellement, si l'implant peut être commandé par tapotement (comme décrit dans le document WO 2013/124362 par exemple), le praticien peut exécuter une série de tapotements selon un code prédéterminé sur la peau du patient en regard de l'implant, qui est détecté par un capteur de l'implant et reconnu comme une commande de validation.

La figure 1A illustre une situation dans laquelle le patient utilise la télécommande 2 pour communiquer avec l'implant 1.

La figure 1B illustre une situation dans laquelle le dispositif de programmation 3 communique avec l'implant 1 par l'intermédiaire de la télécommande 2. Dans ce mode de réalisation, la liaison entre la télécommande 2 et le dispositif de programmation 3 est filaire (schématisée par le fil 4).

La figure 1C illustre également une situation dans laquelle le dispositif de programmation 3 communique avec l'implant 1 par l'intermédiaire de la télécommande 2. Dans ce mode de réalisation, la liaison entre la télécommande 2 et le dispositif de programmation 3 est sans fil (schématisée par les ondes RF2).

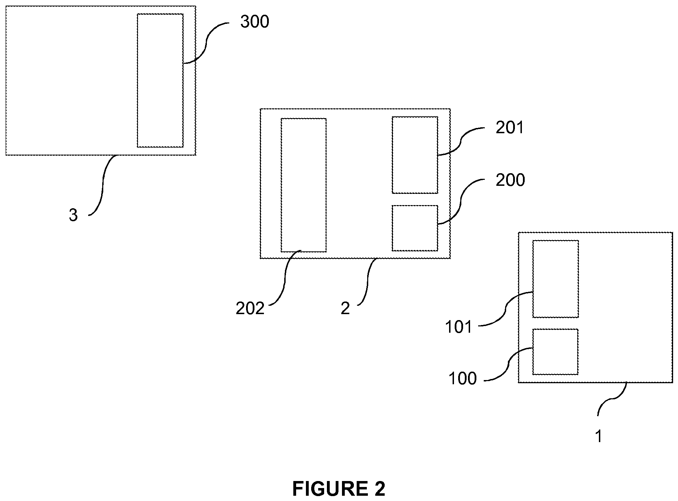

La figure 2 illustre de manière schématique l'architecture générale du système.

### Implant

L'implant peut être tout dispositif médical implantable dans le corps d'un patient, et destiné à communiquer avec des moyens externes au patient. Par exemple, l'implant peut être un sphincter artificiel, un neurostimulateur, etc.

L'implant est pourvu d'un code d'appairage (ou clé secrète) unique, différent des codes d'appairage d'autres implants. Ledit code d'appairage (désigné par le repère 100 sur la figure 2) peut être par exemple mais de manière non limitative un numéro de série, un code à barres (par exemple un QR code), un code NFC (acronyme du terme anglo-saxon « Near Field Communication », c'est-à-dire communication en champ proche), etc. Ce code peut être placé sur l'implant lui-même ou sur l'emballage dans lequel il est livré avant l'implantation, cet emballage étant propre à l'implant.

Ce code d'appairage est adapté pour être lu par le dispositif de programmation si celui-ci comprend un système de lecture adapté pour lire le code d'appairage. De manière alternative, notamment dans le cas où le code d'appairage est constitué de chiffres, de lettres et/ou d'autres signes typographiques, celui-ci peut être saisi manuellement par le praticien au moyen de l'interface utilisateur du dispositif de programmation.

L'implant comprend par ailleurs un émetteur-récepteur radiofréquence (désigné par le repère 101 sur la figure 2) permettant une communication avec la télécommande dans la bande de fréquence RF1. Un tel émetteur-récepteur est connu en lui-même et ne sera donc pas décrit en détail ici, l'homme du métier étant à même de le choisir parmi les émetteurs-récepteurs disponibles sur le marché.

L'implant comprend en outre une mémoire dans laquelle peut être enregistré le code d'appairage de la télécommande, ainsi que des données relatives au patient.

### Télécommande

La télécommande est un dispositif à l'usage du patient, comprenant généralement un ou plusieurs boutons associés à des fonctionnalités particulières.

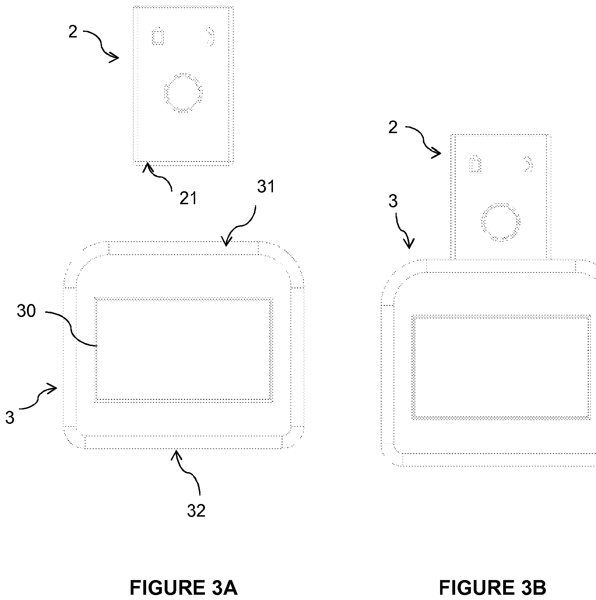

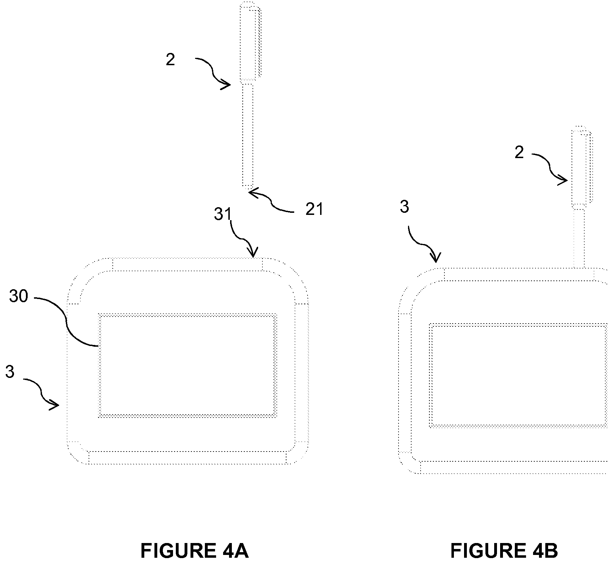

La télécommande peut prendre toute forme adaptée notamment du point de vue ergonomique (facilité d'utilisation, compacité, etc.). Par exemple, pour pouvoir être transportée facilement, la télécommande peut avoir sensiblement le format d'une carte de crédit (cf. figures 3A-3B), ou une forme similaire à celle d'un stylo (cf. figures 4A-4B). Ainsi, elle peut être glissée facilement dans une poche d'un vêtement ou dans un bagage. Naturellement, l'invention n'est pas limitée aux formes illustrées dans les dessins annexés.

La télécommande est pourvue d'un code d'appairage (désigné par le repère 200 sur la figure 2). Ledit code d'appairage peut être par exemple mais de manière non limitative un numéro de série, un code à barres (par exemple un QR code), un code NFC, etc. Ce code peut être placé sur la télécommande elle-même ou sur l'emballage dans lequel elle est livrée avant l'implantation, cet emballage étant propre à la télécommande.

Ce code d'appairage est adapté pour être lu par le dispositif de programmation si celui-ci comprend un système de lecture correspondant au code d'appairage, ou, notamment dans le cas où le code d'appairage est constitué de chiffres, de lettres et/ou d'autres signes typographiques, celui-ci peut être saisi manuellement par le praticien au moyen de l'interface utilisateur du dispositif de programmation.

La télécommande comprend par ailleurs un émetteur-récepteur radiofréquence (désigné par la référence 201 sur la figure 2) permettant une communication avec l'implant dans la bande de fréquence RF1. Un tel émetteur-récepteur est connu en lui-même et ne sera donc pas décrit en détail ici, l'homme du métier étant à même de le choisir parmi les émetteurs-récepteurs disponibles sur le marché.

La télécommande comprend également un microcontrôleur, ainsi qu'un moyen de liaison (désigné par le repère 202 sur la figure 2) avec le dispositif de programmation, dont différents modes de réalisation - filaire ou sans fil - sont décrits plus bas.

La télécommande comprend avantageusement une mémoire dans laquelle peuvent être enregistrées le code d'appairage de l'implant, ainsi que des données récupérées de l'implant.

### Dispositif de programmation

Le dispositif de programmation comprend une interface utilisateur, par l'intermédiaire de laquelle le praticien peut se connecter à la télécommande (ce qui peut nécessiter dans certains cas la saisie d'un mot de passe) et effectuer diverses opérations (programmation ou récupération de données) sur l'implant.

Le dispositif de programmation peut comprendre un lecteur basé sur une technologie adaptée au code d'appairage de l'implant et/ou, le cas échéant, de la télécommande. Par exemple, si le code d'appairage de l'implant est un QR code, le dispositif de programmation comprend un lecteur optique adapté pour lire un tel QR code. Le lecteur peut également être de type RFID ou NFC.

Le dispositif de programmation comprend un microcontrôleur, ainsi qu'un moyen de liaison (désigné par le repère 300 sur la figure 2) avec le dispositif de programmation, dont différents modes de réalisation - filaire ou sans fil - sont décrits plus bas.

De manière avantageuse, le dispositif de programmation se présente sous la forme d'une tablette tactile.

Eventuellement, le dispositif de programmation peut comprendre des moyens de communication de type Wifi ou GSM afin de pouvoir transférer les données recueillies sur un serveur.

### Liaison entre le dispositif de programmation et la télécommande

### Filaire

Selon un mode de réalisation, la liaison entre le dispositif de programmation et la télécommande peut être réalisée par engagement direct de deux connecteurs complémentaires, l'un appartenant au dispositif de programmation, l'autre à la télécommande. Tout type de connectique peut être employé, notamment la connectique USB (acronyme du terme anglo-saxon « Universal Serial Bus »).

Le connecteur du dispositif de programmation est avantageusement disposé dans un logement adapté pour recevoir la télécommande.

Les figures 3A-3B illustrent ainsi un mode de réalisation dans lequel le dispositif de programmation 3 se présente sous la forme d'une tablette comprenant un écran tactile 30 et la télécommande 2 présente une forme rectangulaire plate de type « carte de crédit », comprenant un connecteur 21 sur sa largeur. Une face du dispositif de programmation comprend un logement 31 sous la forme d'une fente de dimension adaptée à la largeur et à l'épaisseur de la télécommande, un connecteur complémentaire (non illustré) de celui de la télécommande étant agencé au fond dudit logement 31. Eventuellement, le dispositif de programmation peut présenter, sur une autre face, un logement 32 pour la télécommande « universelle ». Les figures 3A et 3B présentent le dispositif de programmation 3 et la télécommande 2 respectivement déconnectés et connectés.

Les figures 4A-4B illustrent un mode de réalisation dans lequel le dispositif de programmation 3 se présente sous la forme d'une tablette comprenant un écran tactile 30 et la télécommande 2 présente une forme cylindrique de type « stylo », comprenant un connecteur 21 à une extrémité. Une face du dispositif de programmation comprend un logement 31 sous la forme d'un trou circulaire adapté au diamètre de la télécommande 2, un connecteur complémentaire de celui de la télécommande (non illustré) étant agencé au fond dudit logement 31. Les figures 4A et 4B présentent le dispositif de programmation 3 et la télécommande 2 respectivement déconnectés et connectés.

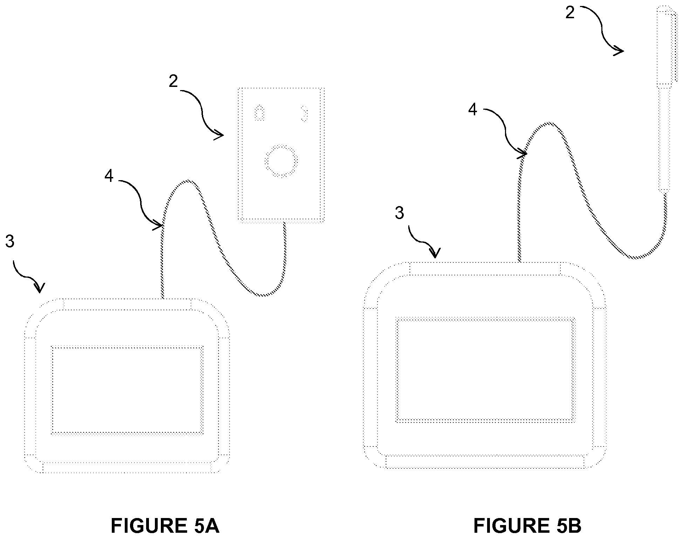

De manière alternative, illustrée sur les figures 5A-5B la liaison entre le dispositif de programmation et la télécommande peut être réalisée au moyen d'un câble électrique 4 branché entre des connecteurs situés respectivement sur le dispositif de programmation 3 et la télécommande 2.

La communication entre la télécommande et le dispositif de programmation peut être réalisée de différentes manières, par exemple :
- entre le microcontrôleur du dispositif de programmation et celui de la télécommande ;
- entre le microcontrôleur du dispositif de programmation et l'émetteur-récepteur de la télécommande ;
- entre l'interface utilisateur du dispositif de programmation et le microcontrôleur de la télécommande ; par exemple, si le dispositif de programmation comprend une interface tactile qui n'est fonctionnelle que lorsque la télécommande est connectée au dispositif de programmation.

L'homme du métier pourra utiliser tout protocole de communication adapté, tel que par exemple mais de manière non limitative : SPI, I2C, RS232, CAB, analogique, etc.

### Sans fil

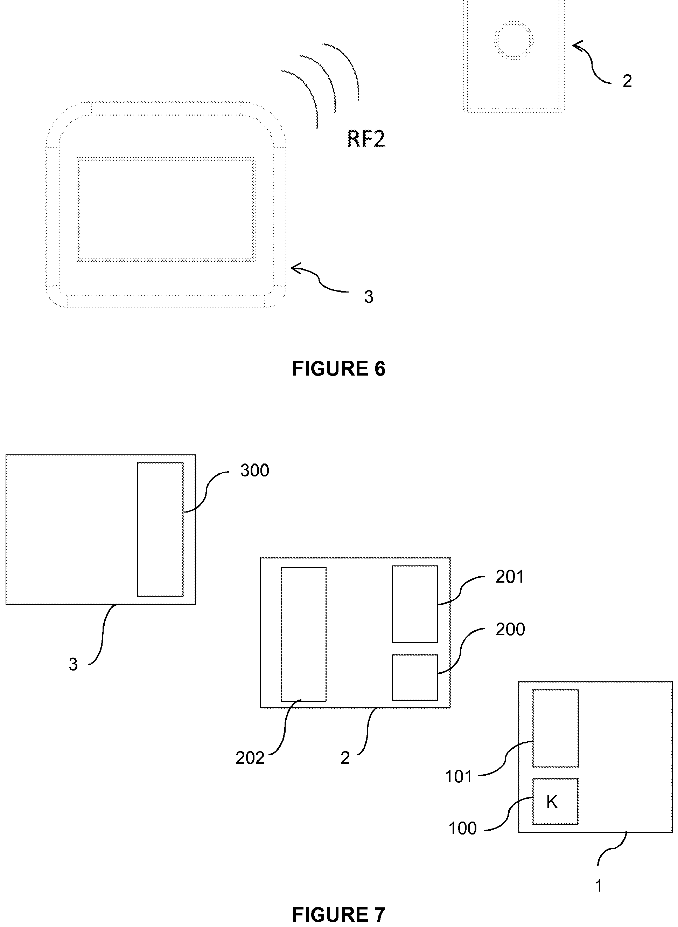

Selon un mode de réalisation illustré sur la figure 6, la télécommande 2 et le dispositif de programmation 3 peuvent comprendre des moyens de liaison sans fil, dans la bande de fréquence RF2.

L'homme du métier pourra utiliser tout protocole de communication de son choix, tel que par exemple mais de manière non limitative : Bluetooth (4.0, BLE, 5, ...), Wifi, Wifi Low Energy, Infrarouge, Zigbee, RF4CE, etc.

Dans le cas d'une liaison sans fil, un appairage entre le dispositif de programmation et la télécommande est nécessaire. L'homme du métier pourra choisir toute technologie d'appairage appropriée, telle que : QR code, Datamatrix, RFID, NFC, iBeacon, Bokode (liste non limitative).

Comme dans le mode de réalisation des figures 3A et 4A, le dispositif de programmation peut comprendre un logement pour l'enfichage de la télécommande, même si dans ce cas le logement ne contient pas de connecteur pour la communication entre la télécommande et le dispositif de programmation, mais sert de support pour la télécommande.

Dans tous les cas (liaison filaire ou sans fil), la liaison entre la télécommande et le dispositif de programmation permet avantageusement d'effectuer un diagnostic de l'état de la télécommande (niveau de charge de la pile de la télécommande, génération d'alarmes en cas de défaillance du système, évaluation de la qualité de communication, récupération de données contenues dans la mémoire de la télécommande, etc.).

Par ailleurs, la connexion de la télécommande au dispositif de programmation peut permettre d'alimenter la télécommande pendant la phase de consultation.

### Procédure de communication

### Avant et/ou pendant l'implantation

Avant et/ou pendant l'implantation, une télécommande destinée au patient est fournie au praticien ou un autre membre du personnel médical. Comme indiqué plus haut, la télécommande et l'implant sont chacun pourvus d'un code d'appairage.

La figure 7 représente l'état du système de la figure 2 avant l'appairage. L'implant 1 est pourvu d'une clé secrète 100 dont la valeur est notée K, la valeur de la clé secrète 200 de télécommande 2 n'étant pas encore définie.

Pour permettre au patient de communiquer ultérieurement avec son implant au moyen de la télécommande, celle-ci doit être appairée avec l'implant, c'est-à-dire que la valeur de la clé secrète de la télécommande doit également être égale à K.

A cet effet, la procédure suivante est mise en oeuvre par l'utilisateur une fois que la télécommande a été connectée au dispositif de programmation.

Dans un premier temps, l'utilisateur sélectionne sur l'interface du dispositif de programmation une demande d'appairage.

Grâce au lecteur du dispositif de programmation, l'utilisateur lit le code d'appairage de la télécommande.

Toujours grâce au lecteur du dispositif de programmation, l'utilisateur lit le code d'appairage de l'implant. Ce code est envoyé à la télécommande par le dispositif de programmation afin de réaliser l'appairage entre la télécommande et l'implant.

Une fois cet appairage validé, une communication est possible entre le dispositif de programmation et l'implant par l'intermédiaire de la télécommande.

L'utilisateur peut alors programmer le fonctionnement de l'implant à partir du dispositif de programmation.

Une fois l'implantation terminée, la télécommande est remise au patient qui la conserve lorsqu'il quitte l'hôpital.

### Après l'implantation (utilisation de la télécommande du patient)

L'utilisateur peut à tout moment communiquer avec l'implant par l'intermédiaire de la télécommande. Tel est notamment le cas s'il souhaite activer ou désactiver une fonction de l'implant, récupérer des informations sur l'état de l'implant (niveau de charge de la batterie le cas échéant, etc.).

Par ailleurs, lors d'une visite chez le praticien, ledit praticien peut également avoir besoin de communiquer avec l'implant, par exemple pour modifier sa programmation, ou pour récupérer des données de l'implant (par exemple, niveau de charge de la batterie, données du patient enregistrées par un ou plusieurs capteurs intégrés à l'implant, etc.).

A cet effet, le praticien dispose du dispositif de programmation.

Dans un premier temps, il doit établir une liaison (filaire ou sans fil, selon le mode de réalisation retenu) entre la télécommande et le dispositif de programmation, et effectuer la demande de communication en lisant le code d'appairage de la télécommande au moyen du lecteur du dispositif de programmation.

Eventuellement, il peut afficher sur l'interface utilisateur des paramètres de l'implant et/ou du patient, lui permettant de vérifier que c'est avec le bon implant que la communication va être établie. Si le praticien confirme la demande de communication, la session de communication entre l'implant et le dispositif de programmation, par l'intermédiaire de la télécommande, démarre.

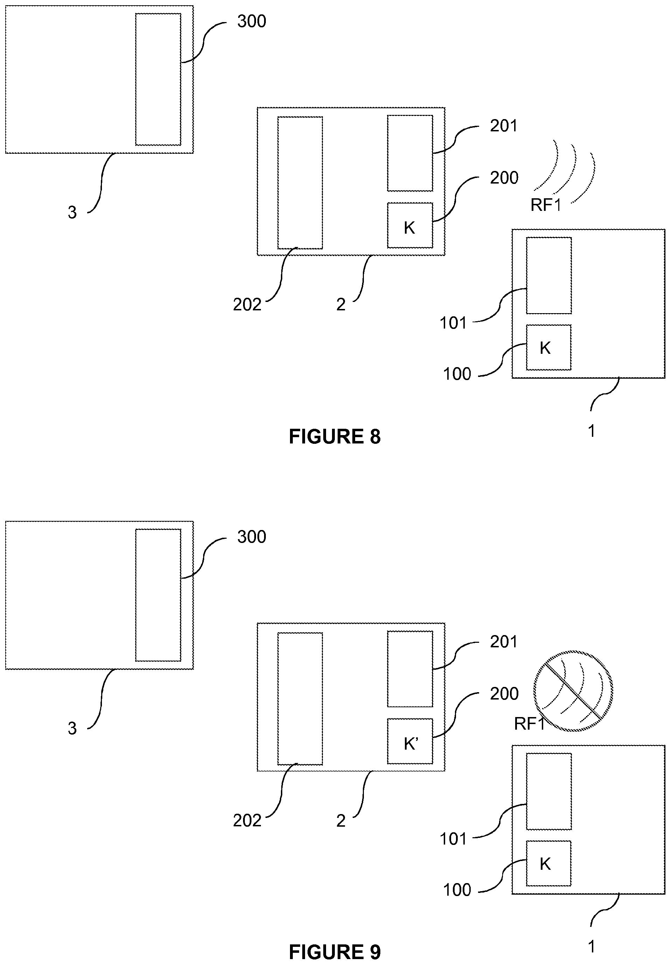

Comme illustré sur la figure 8, c'est l'identité des clés secrètes 200 et 100 qui permet l'établissement d'une communication entre la télécommande 2 et l'implant 1 et, si la télécommande 2 est reliée au dispositif de programmation 3, entre ledit dispositif de programmation 3 et l'implant 1 par l'intermédiaire de la télécommande 2.

En revanche, si, comme illustré sur la figure 9, on tente d'utiliser une télécommande 2 dont la clé secrète 200 présente une valeur K' différente de K, alors aucune communication ne pourra être établie entre la télécommande 2 et l'implant 1, et il sera également impossible d'établir une communication entre le dispositif de programmation 3 et l'implant 1.

### Après l'implantation (utilisation d'une nouvelle télécommande)

Dans certains cas, la télécommande confiée initialement au patient peut ne pas être disponible, par exemple en cas d'oubli ou de perte, ou d'endommagement de la télécommande.

Dans ce cas, une nouvelle télécommande doit être appairée avec l'implant en vue d'être remise au patient.

Deux situations peuvent se présenter.

### Procédure d'appairage normale

Dans la situation la plus simple, le code d'appairage de l'implant est disponible (par exemple, affiché sur l'emballage d'origine, ou conservé dans le dossier du patient).

Dans ce cas, la procédure d'appairage est la suivante.

Dans un premier temps, le praticien sélectionne une demande d'appairage sur l'interface utilisateur du dispositif de programmation.

Le code d'appairage de la télécommande est lu par le lecteur du dispositif de programmation (ou entré manuellement sur l'interface utilisateur). La communication entre la télécommande et le dispositif de programmation est alors établie.

Ensuite, le code d'appairage de l'implant est lu par le lecteur du dispositif de programmation ou entré manuellement par le praticien à l'aide de l'interface du dispositif de programmation. Ce code est alors communiqué à la télécommande pour réaliser l'appairage.

Dans la mesure où l'implant a en mémoire un autre code d'appairage, une demande de confirmation est affichée sur l'interface utilisateur du dispositif de programmation.

Le praticien doit alors valider l'appairage entre la nouvelle télécommande et l'implant, par exemple en saisissant un mot de passe sur l'interface utilisateur, ou en tapotant l'implant selon un code prédéterminé.

### Procédure d'appairage dégradée

Il peut cependant arriver que le code d'appairage de l'implant ne soit plus disponible.

Dans ce cas, le praticien sélectionne une demande d'appairage en mode dégradé sur l'interface utilisateur du dispositif de programmation. De manière avantageuse, l'interface utilisateur affiche une alerte à l'intention du praticien quant à l'utilisation de ce mode d'appairage.

Le code d'appairage de la télécommande est lu par le lecteur du dispositif de programmation. La communication entre la télécommande et le dispositif de programmation est alors établie.

La télécommande détecte les implants situés à sa portée, et l'interface utilisateur affiche des paramètres de chaque implant (par exemple, numéro de série, etc.).

Dans le cas où plusieurs implants sont présents, l'utilisateur sélectionne l'implant souhaité, par exemple en sélectionnant son numéro de série parmi ceux proposés.

Dans la mesure où l'implant a en mémoire un autre code d'appairage, une demande de confirmation est affichée sur l'interface utilisateur du dispositif de programmation.

Le praticien doit alors valider l'appairage entre la nouvelle télécommande et l'implant, par exemple en saisissant un mot de passe sur l'interface utilisateur, ou en tapotant l'implant selon un code prédéterminé.

Les procédures d'appairage susmentionnées sont également applicables pour l'utilisation de la télécommande « universelle » dont dispose le praticien.

## Revendications

1. Système de communication télémétrique pour un dispositif médical implantable (1) comprenant un émetteur-récepteur radiofréquence, comprenant :
- une télécommande (2) adaptée pour être utilisée par un patient dans lequel ledit dispositif médical (1) est implanté, ladite télécommande comprenant un émetteur-récepteur radiofréquence configuré pour communiquer avec ledit dispositif médical implantable dans une première bande de fréquence (RF1) dédiée à des dispositifs médicaux implantables, l'émetteur-récepteur de la télécommande (2) étant appairé avec l'émetteur-récepteur du dispositif médical implantable (1),
- un dispositif de programmation (3) dudit dispositif médical implantable adapté pour être utilisé par un praticien, comprenant une interface utilisateur (30), et configuré pour communiquer avec la télécommande (2) par une liaison filaire ou par une liaison sans fil dans une seconde bande de fréquence (RF2) dédiée à des applications grand public,
le dispositif de programmation (3) étant configuré pour, lorsque ladite liaison est établie entre la télécommande (2) et le dispositif de programmation (3), établir une communication entre le dispositif médical implantable et le dispositif de programmation par l'intermédiaire de la télécommande,
dans lequel :
- le dispositif de programmation (3) comprend un lecteur adapté pour lire un code d'appairage associé au dispositif implantable et/ou un code d'appairage associé à la télécommande, ou
- l'interface utilisateur (30) du dispositif de programmation (3) est adaptée pour permettre une saisie manuelle par le praticien d'un code d'appairage associé au dispositif implantable et/ou d'un code d'appairage associé à la télécommande.

2. Système selon la revendication 1, **caractérisé en ce que** le dispositif de programmation (3) comprend un logement (31) pour l'enfichage de la télécommande (2).

3. Système selon la revendication 1 ou la revendication 2, dans lequel le code d'appairage comprend un code à barres.

4. Système selon l'une des revendications 1 à 3, dans lequel le lecteur est de type optique et/ou communication en champ proche, par exemple de type RFID ou NFC.

5. Système selon l'une des revendications 1 à 4, dans lequel la liaison entre la télécommande (2) et le dispositif de programmation (3) est une liaison sans fil, par exemple de type Bluetooth, Wifi ou infrarouge.

6. Système selon l'une des revendications 1 à 5, **caractérisé en ce que** la liaison entre le dispositif de programmation et la télécommande est une liaison filaire, le dispositif de programmation (3) et la télécommande (2) comprenant des connecteurs respectifs adaptés pour coopérer l'un avec l'autre.

7. Système selon l'une des revendications 1 à 6, **caractérisé en ce que** chacun de la télécommande et du dispositif de programmation comprend un microcontrôleur et **en ce que** la liaison entre la télécommande et le dispositif de programmation comprend une transmission de données entre lesdits microcontrôleurs.

8. Système selon l'une des revendications 1 à 6, **caractérisé en ce que** le dispositif de programmation comprend un microcontrôleur et **en ce que** la liaison entre la télécommande et le dispositif de programmation comprend une transmission de données entre ledit microcontrôleur et l'émetteur-récepteur de la télécommande.

9. Système selon l'une des revendications 1 à 6, **caractérisé en ce que** la télécommande comprend un microcontrôleur et **en ce que** la liaison entre la télécommande et le dispositif de programmation comprend une transmission de données entre ledit microcontrôleur et l'interface utilisateur du dispositif de programmation.

10. Système selon l'une des revendications 1 à 9, **caractérisé en ce que** le dispositif de programmation (3) est configuré pour afficher sur l'interface utilisateur (30) des informations relatives à l'état de la télécommande lorsque la télécommande est en liaison avec ledit dispositif de programmation.

11. Système selon l'une des revendications 1 à 10, **caractérisé en ce que** la liaison entre la télécommande (2) et le dispositif de programmation (3) comprend la fourniture d'énergie à la télécommande par le dispositif de programmation.

12. Procédé de communication entre un dispositif médical implantable (1) comprenant un émetteur-récepteur radiofréquence et un dispositif de programmation (3) d'un système selon l'une des revendications 1 à 11, **caractérisé en ce qu'**il comprend l'établissement d'une liaison entre la télécommande dudit système et le dispositif de programmation et l'établissement d'une communication entre le dispositif de programmation et le dispositif médical implantable par l'intermédiaire de la télécommande dans une bande de fréquence (RF1) dédiée à des dispositifs médicaux implantables.

13. Procédé selon la revendication 12, **caractérisé en ce qu'**il comprend, préalablement à l'établissement de la communication entre le dispositif de programmation (3) et le dispositif médical implantable (2), une étape d'appairage de la télécommande (2) avec le dispositif médical implantable (1) comprenant :
- la lecture d'un code d'appairage de la télécommande (2) par le dispositif de programmation (3), ou la saisie dudit code sur le dispositif de programmation,
- l'établissement d'une liaison entre la télécommande (2) et le dispositif de programmation (3),
- la lecture d'un code d'appairage du dispositif médical implantable (1) par le dispositif de programmation (3), ou la saisie dudit code sur le dispositif de programmation, et l'envoi dudit code à la télécommande (2),
- la validation de l'appairage avec lesdits codes.

14. Procédé selon l'une des revendications 12 ou 13, **caractérisé en ce que**, en l'absence de la télécommande appairée avec le dispositif médical implantable, on utilise une autre télécommande non appairée audit dispositif médical implantable et l'on met en oeuvre les étapes suivantes :
- lecture d'un code d'appairage de ladite autre télécommande par le dispositif de programmation, ou saisie dudit code sur le dispositif de programmation,
- établissement d'une liaison entre ladite autre télécommande et le dispositif de programmation,
- lecture du code d'appairage du dispositif médical implantable, ou la saisie dudit code sur le dispositif de programmation, et envoi dudit code à ladite autre télécommande,
- confirmation, par un utilisateur, d'une commande d'appairage.

15. Procédé selon l'une des revendications 12 ou 13, **caractérisé en ce que**, en l'absence de la télécommande appairée avec le dispositif médical implantable, on utilise une autre télécommande non appairée audit dispositif médical implantable et l'on met en oeuvre les étapes suivantes :
- lecture d'un code d'appairage de ladite autre télécommande par le dispositif de programmation, ou saisie dudit code sur le dispositif de programmation,
- établissement d'une liaison entre ladite autre télécommande et le dispositif de programmation,
- détection d'au moins un dispositif médical à portée de ladite autre télécommande,
- sélection, par un utilisateur, du dispositif médical implantable avec lequel ladite autre télécommande doit être appairée,
- confirmation, par l'utilisateur, d'une commande d'appairage.

16. Procédé selon l'une des revendications 14 ou 15, **caractérisé en ce que** la confirmation de la commande d'appairage avec le dispositif médical implantable (1) sélectionné comprend l'exécution d'une série de tapotements selon un code prédéterminé sur la peau du patient en regard du dispositif médical implantable (1), ledit code étant détecté par un capteur dudit dispositif (1).

17. Procédé selon l'une des revendications 12 ou 13, **caractérisé en ce que**, en l'absence de la télécommande appairée avec le dispositif médical implantable, on utilise une télécommande dite universelle, configurée pour communiquer avec n'importe quel dispositif médical implantable du même type et l'on met en oeuvre les étapes suivantes :
- lecture d'un code d'appairage de ladite télécommande universelle par le dispositif de programmation,
- établissement d'une liaison entre ladite télécommande universelle et le dispositif de programmation,
- détection d'au moins un dispositif médical à portée de ladite télécommande universelle,
- sélection, par un utilisateur, du dispositif médical implantable avec lequel la liaison doit être effectuée,
- confirmation, par l'utilisateur, de la liaison entre ledit dispositif médical implantable et le dispositif de programmation.

18. Procédé selon la revendication 17, **caractérisé en ce que** la confirmation de la liaison entre le dispositif médical implantable (1) sélectionné et le dispositif de programmation comprend l'exécution d'une série de tapotements selon un code prédéterminé sur la peau du patient en regard du dispositif médical implantable (1), ledit code étant détecté par un capteur dudit dispositif (1).

## Patentansprüche

1. Telemetrisches Kommunikationssystem für eine implantierbare medizinische Vorrichtung (1), die einen Hochfrequenz-Transceiver umfasst, umfassend:
- eine Fernsteuerung (2), die dazu ausgelegt ist, von einem Patienten verwendet zu werden, in den die medizinische Vorrichtung (1) implantiert ist, wobei die Fernsteuerung einen Hochfrequenz-Transceiver umfasst, der dazu konfiguriert ist, mit der implantierbaren medizinischen Vorrichtung in einem ersten Frequenzband (RF1) zu kommunizieren, das für implantierbare medizinische Vorrichtungen bestimmt ist, wobei der Transceiver der Fernsteuerung (2) mit dem Transceiver der implantierbaren medizinischen Vorrichtung (1) gekoppelt ist,
- eine Programmiervorrichtung (3) der implantierbaren medizinischen Vorrichtung, die dazu ausgelegt ist, von einem Arzt verwendet zu werden, eine Benutzerschnittstelle (30) umfasst und dazu konfiguriert ist, mit der Fernsteuerung (2) über eine drahtgebundene Verbindung oder über eine drahtlose Verbindung in einem zweiten Frequenzband (RF2) zu kommunizieren, das für Verbraucheranwendungen bestimmt ist,
wobei die Programmiervorrichtung (3) dazu konfiguriert ist, eine Kommunikation zwischen der implantierbaren medizinischen Vorrichtung und der Programmiervorrichtung mittels der Fernsteuerung herzustellen, wenn die Verbindung zwischen der Fernsteuerung (2) und der Programmiervorrichtung (3) hergestellt ist,
wobei:
- die Programmiervorrichtung (3) ein Lesegerät umfasst, das dazu ausgelegt ist, einen der implantierbaren Vorrichtung zugeordneten Kopplungscode und/oder einen der Fernsteuerung zugeordneten Kopplungscode zu lesen, oder
- die Benutzerschnittstelle (30) der Programmiervorrichtung (3) dazu ausgelegt ist, dem Arzt die manuelle Eingabe eines der implantierbaren Vorrichtung zugeordneten Kopplungscodes und/oder eines der Fernsteuerung zugeordneten Kopplungscodes zu ermöglichen.

2. System nach Anspruch 1, **dadurch gekennzeichnet, dass** die Programmiervorrichtung (3) ein Gehäuse (31) zum Einstecken der Fernsteuerung (2) umfasst.

3. System nach Anspruch 1 oder Anspruch 2, wobei der Kopplungscode einen Barcode umfasst.

4. System nach einem der Ansprüche 1 bis 3, wobei das Lesegerät vom optischen Typ und/oder Typ der Nahfeldkommunikation ist, zum Beispiel vom RFID- oder NFC-Typ.

5. System nach einem der Ansprüche 1 bis 4, wobei die Verbindung zwischen der Fernsteuerung (2) und der Programmiervorrichtung (3) eine drahtlose Verbindung ist, zum Beispiel vom Bluetooth-, Wifi- oder Infrarot-Typ.

6. System nach einem der Ansprüche 1 bis 5, **dadurch gekennzeichnet, dass** die Verbindung zwischen der Programmiervorrichtung und der Fernsteuerung eine drahtgebundene Verbindung ist, wobei die Programmiervorrichtung (3) und die Fernsteuerung (2) jeweils Anschlüsse umfassen, die dazu ausgelegt sind, miteinander zusammenzuarbeiten.

7. System nach einem der Ansprüche 1 bis 6, **dadurch gekennzeichnet, dass** die Fernsteuerung und die Programmiervorrichtung jeweils einen Mikrocontroller umfassen und dass die Verbindung zwischen der Fernsteuerung und der Programmiervorrichtung eine Datenübertragung zwischen den Mikrocontrollern umfasst.

8. System nach einem der Ansprüche 1 bis 6, **dadurch gekennzeichnet, dass** die Programmiervorrichtung einen Mikrocontroller umfasst und dass die Verbindung zwischen der Fernsteuerung und der Programmiervorrichtung eine Datenübertragung zwischen dem Mikrocontroller und dem Transceiver der Fernsteuerung umfasst.

9. System nach einem der Ansprüche 1 bis 6, **dadurch gekennzeichnet, dass** die Fernsteuerung einen Mikrocontroller umfasst und dass die Verbindung zwischen der Fernsteuerung und der Programmiervorrichtung eine Datenübertragung zwischen dem Mikrocontroller und der Benutzerschnittstelle der Programmiervorrichtung umfasst.

10. System nach einem der Ansprüche 1 bis 9, **dadurch gekennzeichnet, dass** die Programmiervorrichtung (3) dazu konfiguriert ist, auf der Benutzerschnittstelle (30) Informationen bezüglich des Zustands der Fernsteuerung anzuzeigen, wenn die Fernsteuerung mit der Programmiervorrichtung in Verbindung steht.

11. System nach einem der Ansprüche 1 bis 10, **dadurch gekennzeichnet, dass** die Verbindung zwischen der Fernsteuerung (2) und der Programmiervorrichtung (3) die Energieversorgung der Fernsteuerung durch die Programmiervorrichtung umfasst.

12. Verfahren zur Kommunikation zwischen einer implantierbaren medizinischen Vorrichtung (1), die einen Hochfrequenz-Transceiver umfasst, und einer Programmiervorrichtung (3) eines Systems nach einem der Ansprüche 1 bis 11, **dadurch gekennzeichnet, dass** es das Herstellen einer Verbindung zwischen der Fernsteuerung des Systems und der Programmiervorrichtung und das Herstellen einer Kommunikation zwischen der Programmiervorrichtung und der implantierbaren medizinischen Vorrichtung mittels der Fernsteuerung in einem Frequenzband (RF1), das für implantierbare medizinische Vorrichtungen bestimmt ist, umfasst.

13. Verfahren nach Anspruch 12, **dadurch gekennzeichnet, dass** es vor dem Herstellen der Kommunikation zwischen der Programmiervorrichtung (3) und der implantierbaren medizinischen Vorrichtung (2) einen Schritt des Koppelns der Fernsteuerung (2) mit der implantierbaren medizinischen Vorrichtung (1) umfasst, der Folgendes umfasst:
- Lesen eines Kopplungscodes der Fernsteuerung (2) durch die Programmiervorrichtung (3) oder Eingeben des Codes auf der Programmiervorrichtung,
- Herstellen einer Verbindung zwischen der Fernsteuerung (2) und der Programmiervorrichtung (3),
- Lesen eines Kopplungscodes der implantierbaren medizinischen Vorrichtung (1) durch die Programmiervorrichtung (3) oder Eingeben des Codes auf der Programmiervorrichtung und Senden des Codes an die Fernsteuerung (2),
- Überprüfen der Kopplung mit den Codes.

14. Verfahren nach einem der Ansprüche 12 oder 13, **dadurch gekennzeichnet, dass** bei Abwesenheit der mit der implantierbaren medizinischen Vorrichtung gekoppelten Fernsteuerung eine andere, nicht mit der implantierbaren medizinischen Vorrichtung gekoppelte Fernsteuerung verwendet wird und die folgenden Schritte durchgeführt werden:
- Lesen eines Kopplungscodes der anderen Fernsteuerung durch die Programmiervorrichtung oder Eingeben des Codes auf der Programmiervorrichtung,
- Herstellen einer Verbindung zwischen der anderen Fernsteuerung und der Programmiervorrichtung,
- Lesen des Kopplungscodes der implantierbaren medizinischen Vorrichtung oder Eingeben des Codes auf der Programmiervorrichtung und Senden des Codes an die andere Fernsteuerung,
- Bestätigen eines Kopplungsbefehls durch einen Benutzer.

15. Verfahren nach einem der Ansprüche 12 oder 13, **dadurch gekennzeichnet, dass** bei Abwesenheit der mit der implantierbaren medizinischen Vorrichtung gekoppelten Fernsteuerung eine andere, nicht mit der implantierbaren medizinischen Vorrichtung gekoppelte Fernsteuerung verwendet wird und die folgenden Schritte durchgeführt werden:
- Lesen eines Kopplungscodes der anderen Fernsteuerung durch die Programmiervorrichtung oder Eingeben des Codes auf der Programmiervorrichtung,
- Herstellen einer Verbindung zwischen der anderen Fernsteuerung und der Programmiervorrichtung,
- Erfassen mindestens einer medizinischen Vorrichtung in Reichweite der anderen Fernsteuerung,
- Auswählen der implantierbaren medizinischen Vorrichtung, mit der die andere Fernsteuerung gekoppelt werden muss, durch einen Benutzer,
- Bestätigen eines Kopplungsbefehls durch den Benutzer.

16. Verfahren nach einem der Ansprüche 14 oder 15, **dadurch gekennzeichnet, dass** das Bestätigen des Kopplungsbefehls mit der ausgewählten implantierbaren medizinischen Vorrichtung (1) das Ausführen einer Reihe von Antippberührungen gemäß einem vorbestimmten Code auf der Haut des Patienten, die der implantierbaren medizinischen Vorrichtung (1) zugewandt ist, umfasst, wobei der Code von einem Sensor der Vorrichtung (1) erfasst wird.

17. Verfahren nach einem der Ansprüche 12 oder 13, **dadurch gekennzeichnet, dass** bei Abwesenheit der mit der implantierbaren medizinischen Vorrichtung gekoppelten Fernsteuerung eine Universalfernsteuerung verwendet wird, die dazu konfiguriert ist, mit jeder implantierbaren medizinischen Vorrichtung desselben Typs zu kommunizieren, und die folgenden Schritte durchgeführt werden:
- Lesen eines Kopplungscodes der Universalfernsteuerung durch die Programmiervorrichtung,
- Herstellen einer Verbindung zwischen der Universalfernsteuerung und der Programmiervorrichtung,
- Erfassen mindestens einer medizinischen Vorrichtung in Reichweite der Universalfernsteuerung,
- Auswählen der implantierbaren medizinischen Vorrichtung, mit der die Verbindung hergestellt werden soll, durch einen Benutzer,
- Bestätigen der Verbindung zwischen der implantierbaren medizinischen Vorrichtung und der Programmiervorrichtung durch den Benutzer.

18. Verfahren nach Anspruch 17, **dadurch gekennzeichnet, dass** das Bestätigen der Verbindung zwischen der ausgewählten implantierbaren medizinischen Vorrichtung (1) und der Programmiervorrichtung das Ausführen einer Reihe von Antippberührungen gemäß einem vorbestimmten Code auf der Haut des Patienten, die der implantierbaren medizinischen Vorrichtung (1) zugewandt ist, umfasst, wobei der Code von einem Sensor der Vorrichtung (1) erfasst wird.

## Claims

1. Telemetric communication system for an implantable medical device (1) comprising a radio frequency transceiver, comprising :
- a remote control (2) adapted for use by a patient in whom said medical device (1) is implanted, said remote control comprising a radio frequency transceiver configured to communicate with said implantable medical device in a first frequency band (RF1) dedicated to implantable medical devices, the transceiver of the remote control (2) being paired with the transceiver of the implantable medical device (1),
- a programming device (3) for said implantable medical device adapted for use by a practitioner, comprising a user interface (30), and configured to communicate with the remote control (2) through a wire connection or through a wireless connection in a second frequency band (RF2) dedicated to consumer applications,
the programming device (3) being configured to, when said connection is established between the remote control (2) and the programming device (3), establish communication between the implantable medical device and the programming device via the remote control,
in which :
- the programming device (3) comprises a reader adapted to read a pairing code associated with the implantable device and/or a pairing code associated with the remote control, or
- the user interface (30) of the programming device (3) is adapted to allow manual entry by the practitioner of a pairing code associated with the implantable device and/or a pairing code associated with the remote control.

2. System according to claim 1, **characterized in that** the programming device (3) comprises a housing (31) for plugging in the remote control (2).

3. System according to claim 1 or claim 2, wherein the pairing code comprises a bar code.

4. System according to one of claims 1 to 3, in which the reader is of the optical and/or near-field communication type, for example RFID or NFC.

5. System according to one of claims 1 to 4, in which the connection between the remote control (2) and the programming device (3) is a wireless connection, for example of the Bluetooth, Wifi or infrared type.

6. System according to one of claims 1 to 5, **characterized in that** the connection between the programming device and the remote control is a wired connection, the programming device (3) and the remote control (2) comprising respective connectors adapted to cooperate with each other.

7. System according to one of claims 1 to 6, **characterized in that** each of the remote control and the programming device comprises a microcontroller and **in that** the connection between the remote control and the programming device comprises a data transmission between said microcontrollers.

8. System according to one of claims 1 to 6, **characterized in that** the programming device comprises a microcontroller and **in that** the connection between the remote control and the programming device comprises a data transmission between said microcontroller and the transceiver of the remote control.

9. System according to one of claims 1 to 6, **characterized in that** the remote control comprises a microcontroller and **in that** the connection between the remote control and the programming device comprises a data transmission between said microcontroller and the user interface of the programming device.

10. System according to one of claims 1 to 9, **characterized in that** the programming device (3) is configured to display on the user interface (30) information relating to the state of the remote control when the remote control is connected with said programming device.

11. System according to one of claims 1 to 10, **characterized in that** the connection between the remote control (2) and the programming device (3) comprises supplying power to the remote control by the programming device.

12. Method of communication between an implantable medical device (1) comprising a radio frequency transceiver and a programming device (3) of a system according to one of claims 1 to 11, **characterized in that** it comprises establishing a connection between the remote control of said system and the programming device and establishing communication between the programming device and the implantable medical device via the remote control in a frequency band (RF1) dedicated to implantable medical devices.

13. Method according to claim 12, **characterized in that** it comprises, prior to establishing communication between the programming device (3) and the implantable medical device (2), a step of pairing the remote control (2) with the implantable medical device (1) comprising :
- reading a pairing code of the remote control (2) by the programming device (3), or entering said code on the programming device,
- establishing a connection between the remote control (2) and the programming device (3),
- reading a pairing code of the implantable medical device (1) by the programming device (3), or entering said code on the programming device, and sending said code to the remote control (2),
- validating pairing with said codes.

14. Method according to one of claims 12 or 13, **characterized in that**, in the absence of the remote control paired with the implantable medical device, another remote control not paired with said implantable medical device is used and the following steps are implemented:
- reading a pairing code of said other remote control by the programming device, or entering said code on the programming device,
- establishing a connection between said other remote control and the programming device,
- reading the pairing code of the implantable medical device, or entering said code on the programming device, and sending said code to said other remote control,
- confirming, by a user, a pairing control order.

15. Method according to one of claims 12 or 13, **characterized in that**, in the absence of the remote control paired with the implantable medical device, another remote control not paired with said implantable medical device is used and the following steps are implemented:
- reading a pairing code of said other remote control by the programming device, or entering said code on the programming device,
- establishing a connection between said other remote control and the programming device,
- detecting at least one medical device within range of said other remote control,
- selecting, by a user, the implantable medical device with which said other remote control has to be paired,
- confirming, by a user, a pairing control order.

16. Method according to one of claims 14 or 15, **characterized in that** confirming the pairing control order with the selected implantable medical device (1) comprises performing a series of taps according to a predetermined code on the patient's skin facing the implantable medical device (1), said code being detected by a sensor of said device (1).

17. Method according to one of claims 12 or 13, **characterized in that**, in the absence of the remote control paired with the implantable medical device, a so-called general purpose remote control is used, configured to communicate with any implantable medical device of the same type, and the following steps are implemented:
- reading a pairing code of said general purpose remote control by the programming device,
- establishing a connection between said general purpose remote control and the programming device,
- detecting at least one medical device within range of said general purpose remote control,
- selecting, by a user, the implantable medical device with which the connection must be carried out,
- confirming, by the user, the connection between said implantable medical device and the programming device.

18. Method according to claim 17, **characterized in that** confirming the connection between the selected implantable medical device (1) and the programming device comprises performing a series of taps according to a predetermined code on the patient's skin facing the implantable medical device (1), said code being detected by a sensor of said device (1).
